# EUROPEAN PATENT APPLICATION

(11) **EP 1 374 694 A1**
(43) Date of publication of application: **02.01.2004**
(21) Application number: 03253782.1
(22) Date of filing: 16.06.2003
(51) Int. Cl.: A23K 1/18, C12N 1/16, A23K 1/00, C12N 13/00, A61K 35/66

(54) **Feed additives for animals: Prevention of foot and mouth disease**

(30) Priority: 18.06.2002 US 175016
(71) Applicant: Ultra Biotech Limited, Douglas IM1 1SA, Isle of Man (GB)
(72) Inventor: Cheung Ling Yuk, New Territories (CN)
(74) Representative: Taylor, Kathryn May

(57) **Abstract**

The present invention relates to feed additives for cloven-hooved animals. The invention provides methods for making a biological compositions comprising yeast cells that can improve the immune functions of such animals such that the incidence of foot and mouth disease is reduced. The invention also relates to methods for manufacturing the biological compositions, and methods of using the biological compositions as feed additives.

## Description

### 1. FIELD OF THE INVENTION

The invention relates to biological compositions comprising yeast cells that can improve the immune functions of animals. The invention also relates to methods for manufacturing the biological compositions, and methods of using the biological compositions as animal feed additives.

### 2. BACKGROUND OF THE INVENTION

Foot and Mouth Disease (FMD) is an acute, highly contagious *Picomavirus* infection of cloven-hooved animals. FMD is present in many countries of the world, except for North and Central America (north of Panama), Australia, New Zealand, Great Britain and Scandinavia. The European Union (EU) countries are generally free of FMD. FMD was last reported in 1929 in the U.S.A., 1952 in Canada, and 1954 in Mexico.

The disease is highly contagious and may spread over great distances with movement of infected or contaminated animals, products, objects, and people. Pigs are mainly infected by ingesting infected food. Waste feeding has been associated with outbreaks. Cattle are mainly infected by inhalation, often from pigs, which excrete large amounts of virus by respiratory aerosols and are considered highly important in disease spread. Large amounts of virus are excreted by infected animals before clinical signs are evident, and winds may spread the virus over long distances. People can be infected through skin wounds or the oral mucosa by handling diseased stock, the virus in the laboratory, or by drinking infected milk, but not by eating meat from infected animals. The human infection is temporary and mild. FMD is not considered a public health problem.

The incubation period is 2-21 days (average 3-8) although virus is shed before clinical signs develop. The rate of infection can reach 100%, however mortality can range from 5% (adults) to 75% (suckling pigs and sheep). Recovered cattle may be carriers for 18 to 24 months; sheep for 1 to 2 months. Pigs are not carriers. Clinical signs in cattle are salivation, depression, anorexia and lameness caused by the presence or painful vesicles (blisters) in the skin of the lips, tongue, gums, nostrils, coronary bands, interdigital spaces and teats. Fever and decreased milk production usually precede the appearance of vesicles. The vesicles rupture, leaving large denuded areas which may become secondarily infected. In pigs, sheep and goats the clinical signs are similar but milder. Lameness is the predominant sign.

There is a vaccine available which is rarely used in the European Union, but widely used in some other parts of the world. Vaccination is ruled out mainly for commercial reasons. Although free of symptoms, vaccinated animals, can carry the virus and pass it on to other animals. As a result countries considered free of the disease refuse to import vaccinated livestock. If England decided on vaccination instead of slaughter it would be unable to export livestock to key markets in Europe and the USA. The authorities in England believe the best way of stopping the spread of foot-and-mouth is to destroy any affected herd, incinerate the carcasses and isolate all affected farms inside a five-mile radius exclusion zone. Farms had to be scrubbed with disinfectant twice a day and animals were not allowed on to the land for at least six months after the slaughter.

A major epidemic in England occurred in 1967 and ended in the slaughter of 442,000 animals after more than 2,364 outbreaks were detected. It costed England an estimated £150 million in slaughter costs and lost sales in 1967 and 1968. A total of £27 million was paid out to farmers by the government in compensation. In the recent outbreak of FMD in England which started in February 2001, there were 2030 reported cases as of October 2001, and about 4 million animals were slaughtered to prevent the spread of the disease. Because of the range of species affected, the high rate of infectivity, and the fact that virus is shed before clinical signs occur, FMD is one of the most feared reportable disease in North America. An outbreak of FMD would, and has in the past, cost millions in lost production, loss of export markets, and loss of animals during eradication of the disease. The present invention provides a solution that uses yeasts to improve the immune functions of the animals and hence reduce the incidence and spread of FMD.

For examples of the use of fungal cells and products in animal feed, see the annual Feed Additive Compendium published by The Miller Publishing Company (Minnetonka, Minn.) or the following patent literature:
United States Patent No. 3,903,307 discloses a process for making animal feed that is based on the fermentation of waste molasses or bagasse by yeasts, such as *Candida utilis* and *Trichoderma veride.*
U.S. Patent No. 4,055,667 discloses a liquid animal feed supplement that comprises a colloidal mixture of spent brewer's yeast in an aqueous alcoholic medium.
U.S. Patent No. 4,582,708 discloses an animal feed supplement that comprises live yeast cells *(Saccharomyces* species) having fermenting activity, a texturizing component comprising ground meal, ground legumes or mixtures thereof, a mineral mixture, a liquid binder, a vitamin mixture, and ground montmorillonite.
U.S. Patent No. 5,624,686 discloses an animal feed additive that is prepared by cultivating certain bacteria or yeast species (such as *Saccharomyces cerevisiae, Candida utilis)* and disrupting the microbial cells such that metabolites of the cells are efficiently made available to the animal.
U.S. Patent No. 6,214,337 discloses an animal feed that comprises yeast glucan which is derived from the cell walls of various yeast species (such as *Saccharomyces cerevisiae, Candida utilis).*
Citation of documents herein is not intended as an admission that any of the documents cited herein is pertinent prior art, or an admission that the cited documents are considered material to the patentability of the claims of the present application. All statements as to the date or representations as to the contents of these documents are based on the information available to the applicant and does not constitute any admission as to the correctness of the dates or contents of these documents.

### 3. SUMMARY OF THE INVENTION

The present invention relates to biological compositions that can be added to animal feed to reduce the infection of cloven-hooved animals, such as cattle and swine, by FMD virus.

In one embodiment, the present invention provides biological compositions comprising a plurality of live yeast cells which are capable of improving the immune functions of animals, such as cattle, swine, and sheep, when ingested. The biological compositions can be used for reducing FMD virus infections and the spread of FMD.

In another embodiment, the invention provides methods of making the biological composition. In particular, the methods of the invention comprise culturing yeast cells in the presence of a series of electromagnetic fields of defined frequencies and field strengths, such that the yeast cells becomes metabolically active and potent at stimulating an animal's immune system. Up to four different components of yeast cells can be used to form the biological compositions. The yeast cells can also be subjected to a conditioning or acclimatizing step to improve its performance. The conditioning step comprises culturing the yeast cells in a culture medium comprising gastric juice of an animal and wild hawthorn juice. Methods for manufacturing the biological compositions comprising culturing the yeast cells under activation conditions, mixing various yeast cell cultures of the present invention, followed by drying the yeast cells and packing the final product, are encompassed. In preferred embodiments, the starting yeast cells are commercially available and/or accessible to the public, such as but not limited to *Saccharomyces cerevisiae and Saccharomyces carlsbergensis.*

The biological compositions of the invention can be fed directly to animals or used as an additive to be incorporated into regular animal feed. Animal feed compositions comprising activated yeast cells of the invention and ingredients such as zeolite powder are encompassed by the invention.

### 4. BRIEF DESCRIPTION OF FIGURES

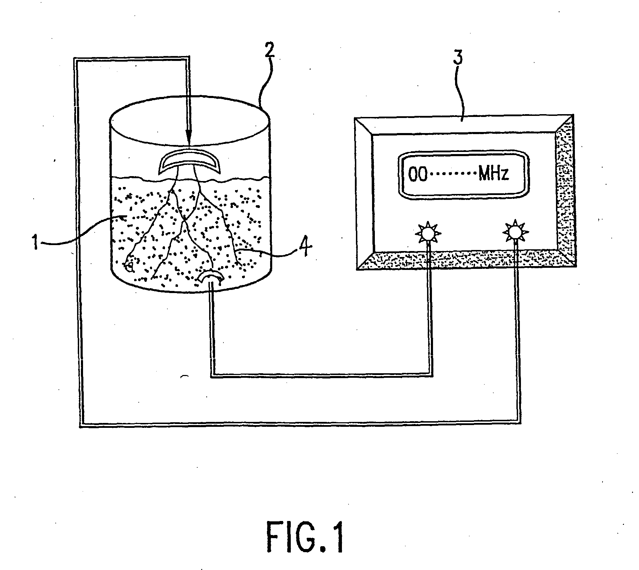

Fig. 1 Activation and conditioning of yeast cells. 1 yeast cell culture; 2 container; 3 electromagnetic field source; 4 electrode.

### 5. DETAILED DESCRIPTION OF THE INVENTION

The present invention relates to biological compositions that can improve the immune functions of animals, and/or reduce the incidence of infections by FMD virus. The present invention provides methods for manufacturing the biological compositions as well as methods for using the biological compositions as animal feed additives.

The biological compositions of the invention comprise yeasts. Unlike the traditional use of yeasts as a component of the feed, the yeast cells of the invention are not a primary source of nutrients for the animals. The yeast cells of the invention serve as a supplement to replace or reduce the antibiotics that are now routinely added to livestock feed. The yeast cells are live when administered orally or ingested along with feed by the animals. While in the gastrointestinal tract of an animal, the yeast cells are capable of stimulating the immune system and improving the immune functions of the animal, thereby reducing the incidence of infectious diseases, especially infections by pathogenic strains of FMD virus. The use of the biological compositions of the invention can reduce the severity of the disease in the animals, and reduce the spread of the FMD virus and hence containing an epidemic.

While the following terms are believed to have well-defined meanings in the art, the following are set forth to define the terms as used herein, and facilitate explanation of the invention.

As used herein, the term "feed" broadly refers to any kind of material, liquid or solid, that is used for nourishing an animal, and for sustaining normal or accelerated growth of an animal including newboms and young developing animals.

The term "animal" as used herein refers to wild and domesticated cloven-hooved animals, and includes Bovidae species, such as but not limited to large ruminants, such as dairy cattle, beef cattle, bisons, and buffalos; as well as swine, sheep, goats, and deer. Also included are animals in Camelidae which include camels, llamas, dromedaries, and vicunas.

The term "immune functions" as used herein broadly encompasses specific and non-specific immunological reactions of the animal, and includes both humoral and cell-mediated defense mechanisms. The immune functions of the animal enable the animal to survive and/or recover from an infection by a pathogen, such as bacteria, viruses, fungi, protozoa, helminths, and other parasites. The immune functions of the animal can also prevent infections, particularly future infections by the same pathogen after the animal had an initial exposure to the pathogen. Many types of immune cells are involved in providing the immune functions, which include various subsets of lymphocytes (e.g., B cells, T cells, NK cells), different types of leukocytes (macrophages, neutrophils, eosinophils, basophils), 5 antigen presenting cells (dendritic cells, endothelial cells) and cells that are found in specialized organs and tissues with immunological activities (bone marrow, lymph nodes, thymus, bursa, Peyer's patch). Details of the immune system of ruminants are described in The Ruminant Immune System by John E. Butler, 1981, Perseus Books, which is incorporated herein by reference in its entirety.

In one embodiment, the present invention provides biological compositions that comprise at least one yeast cell component. Each yeast cell component comprises a population of live yeast cells which have been cultured under a specific set of conditions such that the yeast cells are capable of improving the immune functions of an animal. In preferred embodiments, the biological compositions of the invention comprise up to four yeast cell components.

According to the invention, under certain specific culture conditions, yeasts can be made metabolically active such that they become effective in stimulating and enhancing the immune functions of an animal which ingested the yeasts. Without being bound by any theory or mechanism, the inventor believes that the culture conditions activate 0 and/or amplified the expression of a gene or a set of genes in the yeast cells such that the yeast cells becomes highly potent in stimulating the animal's immune system. It is envisioned that interactions between certain yeast gene products and elements of the animal's immune system is greatly enhanced by the elevated levels of these yeast gene products after the yeast cells have been cultured under the conditions described 5 hereinbelow. These interactions are believed to involve immune cells lining the gastrointestinal tract, lymph nodes, as well as circulating immune cells. As a result of these interactions, the immune functions of the animals, such as responsiveness to and recovery from an infection, and resistance to diseases, are improved. The animals are thus less prone to infection by FMD virus.

As used herein, the term "FMD virus" encompass any strain of FMD virus that causes one or more of the FMD symptoms in cloven-hooved animals. The term includes but is not limited to the seven known serotypes of FMD virus *i.e.,* serotypes A, O, C, SAT1, SAT2, SAT3 and Asia1.

In one embodiment, the biological compositions of the invention can be fed directly to an animal. In another embodiment, the biological compositions can be added to the feed. As known to those skilled in the relevant art, many methods and appliances may be used to mix the biological compositions of the invention with feed. In a particular embodiment, a mixture of culture broths of the yeasts of the present invention are added directly to the feed just prior to feeding the animal. Dried powders of the yeasts can also be added directly to the feed just prior to feeding the animal. In yet another embodiment of the present invention, the yeast cells are mixed with the raw constituents of the feed with which the yeast cells become physically incorporated. The biological compositions may be applied to and/or mixed with the feed by any mechanized means which may be automated.

The amount of biological composition used depends in part on the feeding regimen and the type of feed, and can be determined empirically. For example, the useful ratio of biological composition to animal feed ranges from 0.1% to 1% by dry weight, preferably, 0.3 to 0.8%, and most preferably at about 0.5%. Although not necessary, the biological compositions of the invention can also be used in conjunction or in rotation with other types of supplements, such as but not limited to vitamins, minerals, and vaccines.

Described below in Section 5.1 and 5.2 are four yeast cell components of the invention and methods of their preparation. Section 5.3 describes the manufacture of the biological compositions of the invention which comprises at least one of the four yeast cell components.

### 5.1. PREPARATION OF THE YEAST CELL CULTURES

The present invention provides yeast cells that are capable of improving the immune functions of an animal which ingested the yeast cells. Up to four different yeast cell components can be combined to make the biological compositions.

A yeast cell component of the biological composition is produced by culturing a plurality of yeast cells in an appropriate culture medium in the presence of an alternating electromagnetic field or multiple alternating electromagnetic fields in series over a period of time. The culturing process allows yeast spores to germinate, yeast cells to grow and divide, and can be performed as a batch process or a continuous process. As used herein, the terms "alternating electromagnetic field", "electromagnetic field" or "EM field" are synonymous. An electromagnetic field useful in the invention can be generated by various means well known in the art. A schematic illustration of exemplary setups are depicted respectively in Fig. 1. An electromagnetic field of a desired frequency and a desired field strength is generated by an electromagnetic wave source (3) which comprises one or more signal generators that are capable of generating electromagnetic waves, preferably sinusoidal waves, and preferably in the frequency range of 8000 MHz - 18000 MHz. Such signal generators are well known in the art. Signal generators capable of generating signal with a narrower frequency range can also be used. If desirable, a signal amplifier can also be used to increase the output signal, and thus the strength of the EM field.

The electromagnetic field can be applied to the culture by a variety of means including placing the yeast cells in close proximity to a signal emitter connected to a source of electromagnetic waves. In one embodiment, the electromagnetic field is applied by signal emitters in the form of electrodes that are submerged in a culture of yeast cells (1). In a preferred embodiment, one of the electrodes is a metal plate which is placed on the bottom of a non-conducting container (2), and the other electrode comprises a plurality of wires or tubes so configured inside the container such that the energy of the electromagnetic field can be evenly distributed in the culture. For an upright culture vessel, the tips of the wires or tubes are placed within 3 to 30 cm from the bottom of the vessel (i.e, approximately 2 to 10% of the height of the vessel from the bottom). The number of electrode wires used depends on both the volume of the culture and the diameter of the wire. For example, for a culture having a volume of 10 liter or less, two or three electrode wires having a diameter of between 0.5 to 2.0 mm can be used. For a culture volume of 10 liter to 100 liter of culture, the electrode wires or tubes can have a diameter of 3.0 to 5.0 mm. For a culture volume of 100 liter to 1000 liter, the electrode wires or tubes can have a diameter of 6.0 to 15.0 mm. For a culture having a volume greater than 1000 liter, the electrode wires or tubes can have a diameter of between 20.0 to 25.0 mm.

In various embodiments, yeasts of the genera of *Saccharomyces, Candida, Crebrothecium, Geotrichum, Hansenula, Kloeckera, Lipomyces, Pichia, Rhodosporidium, Rhodotorula Torulopsis, Trichosporon,* and *Wickerhamia* can be used in the invention.

Non-limiting examples of yeast strains include *Saccharomyces cerevisiae* Hansen, ACCC2034, ACCC2035, ACCC2036, ACCC2037, ACCC2038, ACCC2039, ACCC2040, ACCC2041, ACCC2042, AS2.1, AS2.4, AS2.11, AS2.14, AS2.16, AS2.56, AS2.69, AS2.70, AS2.93, AS2.98, AS2.101, AS2.109, AS2.110, AS2.112, AS2.139, AS2.173, AS2.174, AS2.182, AS2.196, AS2.242, AS2.336, AS2.346, AS2.369, AS2.374, AS2.375, AS2.379, AS2.380, AS2.382, AS2.390, AS2.393, AS2.395, AS2.396, AS2.397, AS2.398, AS2.399, AS2.400, AS2.406, AS2.408, AS2.409, AS2.413, AS2.414, AS2.415, AS2.416, AS2.422, AS2.423, AS2.430, AS2.431, AS2.432, AS2.451, AS2.452, AS2.453, AS2.458, AS2.460, AS2.463, AS2.467, AS2.486, AS2.501, AS2.502, AS2.503, AS2.504, AS2.516, AS2.535, AS2.536, AS2.558, AS2.560, AS2.561, AS2.562, AS2.576, AS2.593, AS2.594, AS2.614, AS2.620, AS2.628, AS2.631, AS2.666, AS2.982, AS2.1190, AS2.1364, A52.1396, IFFI 1001, IFFI 1002, IFFI 1005, IFFI 1006, IFFI 1008, IFFI 1009, IFFI 1010, IFFI 1012, IFFI 1021, IFF 1027, IFFI 1037, IFFI 1042, IFFI 1043, IFFI 1045, IFFI 1048, IFFI 1049, IFFI 1050, EFFI 1052, IFFI 1059, IFFI 1060, IFFI 1063, IFFI 1202, IFFI 1203, IFFI 1206, IFFI 1209, IFFI 1210, IFFI 1211, IFFI 1212, IFFI 1213, IFFI 1215, IFFI 1220, IFFI 1221, IFFI 1224, IFFI 1247, IFFI 1248, IFFI 1251, IFFI 1270, IFFI 1277, IFFI 1287, IFFI 1289, IFFI 1290, IFFI 1291, IFFI 1292, IFFI 1293, IFFI 1297, IFFI 1300, IFFI 1301, IFFI 1302, IFFI 1307, IFFI 1308, IFFI 1309, EFFI 1310, IFFI 1311, IFFI 1331, IFFI 1335, IFFI 1336, IFFI 1337, IFFI 1338, IFFI L339, IFFI 1340, IFFI 1345, IFFI 1348, IFFI 1396, IFFI 1397, IFFI 1399, IFFI 1411, IFFI 1413; *Saccharomyces cerevisiae* Hansen Var. ellipsoideus (Hansen) Dekker, ACCC2043, AS2.2, AS2.3, AS2.8, AS2.53, AS2.163, AS2.168, AS2.483, AS2.541, AS2.559, AS2.606, AS2.607, AS2.611. AS2.612; *Saccharomyces chevalieri* Guillermond, AS2.131, AS2.213; *Saccharomyces delbrueckii,* AS2.285; *Saccharomyces delbrueckii* Lindner var. mongolicus Lodder et van Rij, AS2.209, AS2.1157; *Saccharomyces exiguous* Hansen, AS2.349, AS2.1158; *Saccharomyces fermentati* (Saito) Lodder et van Rij, AS2.286, AS2.343; *Saccharomyces logos* van laer et Denamur ex Jorgensen, AS2.156, AS2.327, AS2.335; *Saccharomyces mellis* Lodder et Kreger Van Rij, AS2.195; *Saccharomyces microellipsoides* Osterwalder, AS2.699; Saccharomyces *oviformis* Osterwalder, AS2.100; *Saccharomyces rosei* (Guilliermond) Lodder et kreger van Rij, AS2.287; *Saccharomyces rouxii* Boutroux, AS2.178, AS2.180, AS2.370, AS2.371; *Saccharomyces sake* Yabe, ACCC2045; *Candida arborea,* AS2.566; *Candida Krusei* (Castellani) Berkhout, AS2.1045; *Candida* lambica(Lindner et Genoud) van.Uden et Buckley, AS2.1182; *Candida lipolytica* (Harrison) Diddens et Lodder, AS2.1207, AS2.1216, AS2.1220, AS2.1379, AS2.1398, AS2.1399, AS2.1400; *Candida parapsilosis* (Ashford) Langeron et Talice, AS2.590; *Candida parapsilosis* (Ashford) et Talice Var. intermedia Van Rij et Verona, AS2.491; *Candida pulcherriman* (Lindner) Windisch, AS2.492; *Candida rugousa* (Anderson) Diddens et Loddeer, AS2.511, AS2.1367, AS2.1369, AS2.1372, AS2.1373, AS2.1377, AS2.1378, AS2.1384; *Candida tropicalis* (Castellani) Berkout, ACCC2004, ACCC2005, ACCC2006, AS2.164, AS2.402, AS2.564, AS2.565, AS2.567, AS2.568, AS2.617, AS2.1387; *Candida utilis* Henneberg Lodder et Kreger Van Rij, AS2.120, AS2.281, AS2.1180; *Crebrothecium ashbyii* (Guillermond) Routein, AS2.481, AS2.482, AS2.1197; *Geotrichum candidum* Link, ACCC2016, AS2.361, AS2.498, AS2.616, AS2.1035, AS2.1062, AS2.1080, AS2.1132, AS2.1175, AS2.1183; *Hansenula anomala* (Hansen) H et P sydow, ACCC2018, AS2.294, AS2.295, AS2.296, AS2.297, AS2.298, AS2.299, AS2.300, AS2.302, AS2.338, AS2.339, AS2.340, AS2.341, AS2.470, AS2.592, AS2.641, AS2.642, AS2.635, AS2.782, AS2.794; *Hansenula arabitolgens* Fang, AS2.887; *Hansenula jadinii* Wickerham, ACCC2019; *Hansenula saturnus* (Klocker) H et P sydow, ACCC2020; *Hansenula schneggii* (Weber) Dekker, AS2.304; *Hansenula subpelliculosa* Bedford, AS2.738, AS2.740, AS2.760, AS2.761, AS2.770, AS2.783, AS2.790, AS2.798, AS2.866; *Kloeckera apiculata* (Reess emend. Klocker) Janke, ACCC2021, ACCC2022, ACCC2023, AS2.197, AS2.496, AS2.711, AS2.714; *Lipomyces starkeyi* Lodder et van Rij, ACCC2024, AS2.1390; *Pichia farinosa* (Lindner) Hansen, ACCC2025, ACCC2026, AS2.86, AS2.87, AS2.705, AS2.803; *Pichia membranaefaciens* Hansen, ACCC2027, AS2.89, AS2.661, AS2.1039; *Rhodosporidium toruloides* Banno, ACCC2028; *Rhodotorula glutinis* (Fresenius) Harrison, ACCC2029, AS2.280, ACCC2030, AS2.102, AS2.107, AS2.278, AS2.499, AS2.694, AS2.703, AS2.704, AS2.1146; *Rhodotorula minuta* (Saito) Harrison, AS2.277; *Rhodotorula rubar* (Demme) Lodder, ACCC2031, AS2.21, AS2.22, AS2.103, AS2.105, AS2.108, AS2.140, AS2.166, AS2.167, AS2.272, AS2.279, AS2.282; *Saccharomyces carlsbergensis* Hansen, AS2.113, ACCC2032, ACCC2033, AS2.312, AS2.116, AS2.118, AS2.121, AS2.132, AS2.162, AS2.189, AS2.200, AS2.216, AS2.265, AS2.377, AS2.417, AS2.420, AS2.440, AS2.441, AS2.443, AS2.444, AS2.459, AS2.595, AS2.605, AS2.638, AS2.742, AS2.745, AS2.748, AS2.1042; *Saccharomyces uvarum Beijer,* IFFI 1023, IFFI 1032, IFFI 1036, IFFI 1044, IFFI 1072, IFFI 1205, IFFI 1207; *Saccharomyces willianus* Saccardo, AS2.5, AS2.7, AS2.119, AS2.152, AS2.293, AS2.381, AS2.392, AS2.434, AS2.614, AS2.1189; *Saccharomyces sp.,* AS2.311; *Saccharomyces ludwigii* Hansen, ACCC2044, AS2.243, AS2.508; *Saccharomyces sinenses* Yue, AS2.1395; *Schizosaccharomyces octosporus* Beijerinck, ACCC 2046, AS2.1148; *Schizosaccharomyces pombe* Linder, ACCC2047, ACCC2048, AS2.248, AS2.249, AS2.255, AS2.257, AS2.259, AS2.260, AS2.274, AS2.994, AS2.1043, AS2.1149, AS2.1178, IFFI 1056; *Sporobolomyces roseus* Kluyver et van Niel, ACCC 2049, ACCC 2050, AS2.619, AS2.962, AS2.1036, ACCC2051, AS2.261, AS2.262; *Torulopsis candida* (Saito) Lodder, ACCC2052, AS2.270; *Torulopsis famta* (Harrison) Lodder et van Rij, ACCC2053, AS2.685; *Torulopsis globosa* (Olson et Hammer) Lodder et van Rij, ACCC2054, AS2.202; *Torulopsis inconspicua* Lodder et van Rij, AS2.75; *Trichosporon behrendii* Lodder et Kreger van Rij, ACCC2055, AS2.1193; *Trichosporon capitatum* Diddens et Lodder, ACCC2056, AS2.1385; *Trichosporon cutaneum(de* Beurm et al.)Ota, ACCC2057, AS2.25, AS2.570, AS2.571, AS2.1374; *Wickerhamia fluoresens* (Soneda) Soneda, ACCC2058, AS2.1388. Yeasts of the *Saccharomyces* genus are generally preferred. Among strains of *Saccharomyces cerevisiae, Saccharomyces cerevisiae* Hansen is a preferred strain.

Generally, yeast strains useful for the invention can be obtained from private or public laboratory cultures, or publically accessible culture deposits, such as the American Type Culture Collection, 10801 University Boulevard, Manassas, VA 20110-2209 and the China General Microbiological Culture Collection Center (CGMCC), China Committee for Culture Collection of Microorganisms, Institute of Microbiology, Chinese Academy of Sciences, Haidian, P.O. Box 2714, Beijing, 100080, China.

Although it is preferred, the preparation of the yeast cell components of the invention is not limited to starting with a pure strain of yeast. Each yeast cell component may be produced by culturing a mixture of yeast cells of different species or strains. The constituents of a yeast cell component can be determined by standard yeast identification techniques well known in the art.

In various embodiments of the invention, standard techniques for handling, transferring, and storing yeasts are used. Although it is not necessary, sterile conditions or clean environments are desirable when carrying out the manufacturing processes of the invention. Standard techniques for handling animal blood and immune cells, and for studying immune functions of an animal are also used. Details of such techniques are described in Advances in Laboratory Methods: General Haematology, 2000, Assendelft et al., (Ed.), Arnold, Edward (Publisher); Handbook of Vertebrate Immunology, 1998, Pastoret et al. (Ed.), Academic Press, and Current Protocols In Immunology, 1991, Coligan, et al. (Ed), John Wiley & Sons, Inc., which are both incorporated herein by reference in their entireties.

In one embodiment, the yeast cells of the first yeast cell component are cultured in the presence of at least one alternating electromagnetic (EM) field with a frequency in the range of 9205 MHz to 9230 MHz, preferably 9208 to 9230 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 9208, 9209, 9210, 9211, 9212, 9213, 9214, 9215, 9216, 9217, 9218, 9219, 9220, 9221, 9222, 9223, 9224, 9225, 9226, 9227, 9228, 9229 or 9230 MHz. The field strength of the EM field(s) is in the range of 50 to 250 mV/cm. The yeast cells can be cultured in the EM fields for 42 to 134 hours. The yeast culture can remain in the same container and use the same set of electromagnetic wave generator and emitters when switching from one EM field to another EM field.

The culture process can be initiated by inoculating 1000ml of medium with an inoculum of the selected yeast strain(s) of about 10⁸ cells. The starting culture is kept at 33±5 °C for 24 to 56 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 1 provides an exemplary medium for culturing the first yeast cell component of the invention.

**Table 1**

| Medium Composition | Quantity |
|---|---|
| Soluble starch | 8g |
| Glycose | 15g |
| K₂HPO₄ | 0.2g |
| MgSO₄• 7H₂O | 0.3g |
| NaCl | 0.25g |
| CaSO₄•2H₂O | 0.2g |
| CaCO₃•5H₂O | 3.0g |
| Peptone | 1.2g |
| Animal serum | 300ml |
| Autoclaved water | 700ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃,MgSO₄, NaCl, and CaSO₄.

The animal serum is a fraction of blood that comprises white blood cell, and can be prepared from whole blood (1000-2000 ml) by standard methods known in the art, such as density gradient centrifugation. Without limitation and for convenience, either bovine or porcine serum can be used. Red blood cells are separated and discarded. The serum may be diluted or concentrated. The serum is added to the culture medium after the medium has been autoclaved and cooled to about 45 °C.

It should be noted that the composition of the media provided in Table 1 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM fields) for an extended period of time (e.g., one or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a first yeast cell component of the invention with *Saccharomyces carlsbergensis* strain AS2.441 is provided in Section 6 hereinbelow. *Saccharomyces cerevisiae* strain AS2.558 is also preferred for making the first yeast cell component.

In another embodiment, the yeast cells of the second yeast cell component are cultured in the presence of at least one alternating electromagnetic (EM) field with a frequency in the range of 8135 MHz to 8155 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 8135, 8136, 8137, 8138, 8139, 8140, 8141, 8142, 8143, 8144, 8145, 8146, 8147, 8148, 8149, 8150, 8151, 8152, 8153, 8154 or 8155 MHz. The field strength of the EM field(s) is in the range of 45 to 220 mV/cm. The yeast cells can be cultured in the EM fields for 48 to 140 hours. The yeast culture can remain in the same container and use the same set of electromagnetic wave generator and emitters when switching from one EM field to another EM field.

The culture process can be initiated by inoculating 1000ml of medium with an inoculum of the selected yeast strain(s) of about 10⁸ cells. The starting culture is kept at 33±5 °C for 24 to 56 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 2 provides an exemplary medium for culturing the second yeast cell component of the invention.

**Table 2**

| Medium Composition | Quantity |
|---|---|
| Soluble starch | 12g |
| Sucrose | 10g |
| K₂HPO₄ | 0.25g |
| MgSO₄•7H₂O | 0.2g |
| NaCl | 0.30g |
| CaSO₄•2H₂O | 0.1 g |
| CaCO₃• 5H₂O | 4.0g |
| Yeast extract | 0.4g |
| Animal serum | 350ml |
| Medium Composition | Quantity |
| Autoclaved water | 650ml |

in general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 2 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the second yeast cell component of the invention may be recovered from the culture by various methods known in the art, and stored at a temperature below about 0°C to 4°C. The recovered yeast cells may also be dried and stored in powder form.

A non-limiting example of making a second yeast cell component of the invention with *Saccharomyces carlsbergensis* strain AS2.162 is provided in Section 6 hereinbelow. *Saccharomyces cerevisiae* strain AS2.182 is also preferred for making the second yeast cell component.

In yet another embodiment, the yeast cells of the third yeast cell component are cultured in the presence of at least one alternating electromagnetic (EM) field with a frequency in the range of 11310 MHz to 11330 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 11310, 11311, 11312, 11313, 11314, 11315, 11316, 11317,11318, 11319,11320, 11321, 11322, 11323, 11324, 11325, 11326, 11327, 11328, 11329, or 11330 MHz.

The field strength of the EM fields) is in the range of 63 to 220 mV/cm. The yeast cells can be cultured in the EM fields for 36 to 140 hours. The yeast culture can remain in the same container and use the same set of electromagnetic wave generator and emitters when switching from one EM field to another EM field.

The culture process can be initiated by inoculating 1000ml of medium with an inoculum of the selected yeast strain(s) of about 10⁸ cells. The starting culture is kept at 33±5°C for 24 to 56 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 3 provides an exemplary medium for culturing the third yeast cell component of the invention.

**Table 3**

| Medium Composition | Quantity |
|---|---|
| Glycerol | 18.0g |
| K₂HPO₄ | 0.3g |
| MgSO₄•7H₂O | 0.30g |
| NaCl | 0.30g |
| CaSO₄•₂H₂O | 0.40g |
| CaCO₃•5H₂O | 4.0g |
| Peptone | 1.5g |
| Animal serum | 300ml |
| Autoclaved water | 700ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 3 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the third yeast cell component of the invention may be recovered from the culture by various methods known in the art, and stored at a temperature below about 0°C to 4°C. The recovered yeast cells may also be dried and stored in powder form.

A non-limiting example of making a third yeast cell component of the invention with *Saccharomyces carlsbergensis* strain AS2.189 is provided in Section 6 hereinbelow. *Saccharomyces cerevisiae* strain AS2.14 is also preferred for making the third yeast cell component.

In yet another embodiment, the yeast cells of the fourth yeast cell component are cultured in the presence of at least one alternating electromagnetic (EM) field with a frequency in the range of 17020 MHz to 17040 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 17020, 17021, 17022, 17023, 17024, 17025, 17026, 17027, 17028, 17029, 17030, 17031, 17032, 17033, 17034, 17035, 17036, 17037, 17038, 17039, or 17040 MHz. The field strength of the EM fields) is in the range of 50 to 230 mV/cm. The yeast cells can be cultured in the EM fields for 42 to 130 hours. The yeast culture can remain in the same container and use the same set of electromagnetic wave generator and emitters when switching from one EM field to another EM field.

The culture process can be initiated by inoculating 1000ml of medium with an inoculum of the selected yeast strain(s) of about 10⁸ cells. The starting culture is kept at 33±5 ° C for 24 to 56 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 4 provides an exemplary medium for culturing the fourth yeast cell component of the invention.

**Table 4**

| Medium Composition | Quantity |
|---|---|
| Glycerol | 18.0g |
| K₂HPO₄ | 0.22g |
| MgSO₄•7H₂O | 0.2g |
| NaCl | 0.23g |
| CaSO₄•2H₂O | 0.23g |
| CaCO₃•5H₂O | 2.5g |
| Peptone | 1.5g |
| Bovine serum | 200ml |
| Autoclaved water | 800ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 4 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the fourth yeast cell component of the invention may be recovered from the culture by various methods known in the art, and stored at a temperature below about 0°C to 4°C. The recovered yeast cells may also be dried and stored in powder form.

A non-limiting example of making a fourth yeast cell component of the invention with *Saccharomyces carlsbergensis* strain AS2.638 is provided in Section 6 hereinbelow. Another *Saccharomyces cerevisiae* strain ACCC2036 is also preferred for making the fourth yeast cell component.

### 5.2. CONDITIONING OF THE YEAST CELLS

According to the invention, performance of the activated yeast cells can be optimized by culturing the activated yeast cells in the presence of an extract from the stomach (e.g., the gastric juice) of a cloven-hoofed animal. The inclusion of this additional conditioning or acclimatizing process allows the activated yeast cells to adapt to and endure the acidic environment of the animal's stomach. The method for conditioning or acclimatizing activated yeast cells of the invention comprises culturing yeast cells in the presence of gastric juice of an animal in an alternating electromagnetic (EM) field.

The culture process can be initiated by inoculating 1000ml of a conditioning medium with about 10 ml of activated yeasts containing about 10⁸ cells/ml (as prepared by the methods described in section 5.1). The initial number of yeast cells in the conditioning medium is about 10⁶ cells/ml. An equivalent number of dried yeast cells can also be used as an inoculum. The conditioning medium comprises per 1000 ml about 600 ml of gastric juice of an animal and about 400 ml of wild hawthorn juice. The process can be scaled up or down according to needs.

The gastric juice of a cattle or a pig can be obtained from the stomach content of a freshly slaughtered animal. Although not essential, the animal is preferably kept under a clean environment, and fed a standard diet, preferably germ-free. The gastric juice can be separated by centrifugation or filtration of the stomach content to remove debris and/or microorganisms. The gastric juice so obtained can be stored at 4°C. Preferably, the collection procedures and storage are carried out under sterile conditions.

The wild hawthorn juice is an extract of wild hawthorn fruits prepared by drying the fruits to less than about 8% moisture, crushing the dried fruits to less than about 20 mesh, and mixing 5 ml of water per 1 gram of the crushed wild hawthorn. The mixture is then allowed to settle without stirring for 6 hours, and the clear liquid above is collected for use as the wild hawthorn juice used in the conditioning process. Other methods that can be used to collect the hawthorn juice include centrifugation or filtration of the mixture. Preferably, the collection procedures and storage are carried out under sterile conditions.

In one embodiment, the activated yeast cells of the first yeast cell component are cultured in conditioning medium in the presence of a series of two EM fields. The frequencies of the EM fields are 9211, and 9217 MHz. The field strength is in the range of 60 to 250 mV/cm, preferably at 187 ± 1 mV/cm. About 10⁹ activated yeast cells of the first yeast cell component are added to 1000 ml of conditioning medium. The temperature is maintained at 36 ± 2°C. The yeast culture is exposed to the EM field for about 42-134 hours.

In another embodiment, the activated yeast cells of the second yeast cell component are cultured in conditioning medium in the presence of a series of two EM fields. The frequencies of the EM fields are 8144 and 8153 MHz. The field strength is in the range of 60 to 250 mV/cm, preferably at 187 ± 1 mV/cm. About 10⁹ activated yeast cells of the second yeast cell component are added to 1000 ml of conditioning medium. The temperature is maintained at 36 ± 2°C. The yeast culture is exposed to the EM field for about 48-140 hours.

In yet another embodiment, the activated yeast cells of the third yeast cell component are cultured in conditioning medium in the presence of a series of two EM fields. The frequencies of the EM fields are 11321 and 11328 MHz. The field strength is in the range of 60 to 250 mV/cm, preferably at 187 ± 1 mV/cm. About 10⁹ activated yeast cells of the third cell component are added to 1000 ml of conditioning medium. The temperature is maintained at 36 ± 2°C. The yeast culture is exposed to the EM field for about 36-104 hours.

In yet another embodiment, the activated yeast cells of the fourth yeast cell component are cultured in conditioning medium in the presence of a series of two EM fields. The frequencies of the EM fields are 17021 and 17025 MHz. The field strength is in the range of 60 to 250 mV/cm, preferably at 187 ± 1 mV/cm. About 10⁹ activated yeast cells of the first yeast cell component are added to 1000 ml of conditioning medium. The temperature is maintained at 36 ± 2°C. The yeast culture is exposed to the EM field for about 42-130 hours.

The activated and conditioned yeast cells of all four yeast cell components may be recovered from the culture by various methods known in the art, and preferably stored in powder form at a temperature below about 0°C to 4°C. Preferably, the powder form of the yeast cells comprises greater than about 10¹⁰ yeast cells per gram. The activated and conditioned yeast cells can be dried as follows: the yeast cells was dried in a first dryer at a temperature not exceeding 60 ± 2 °C for a period of 5 minutes so that yeast cells quickly became dormant. The yeast cells were then sent to a second dryer and dried at a temperature not exceeding 65 ± 2 °C for a period of about 8 minutes to further remove water. The dried yeast cells were then cool to room temperature.

The activated and conditioned yeast cells can be used separately but preferably as a mixture in a biological composition, or used as a starter culture for large scale manufacturing.

### 5.3 MANUFACTURE OF THE BIOLOGICAL COMPOSITIONS

The present invention also encompasses methods of manufacturing of the biological compositions of the invention. The activated and conditioned yeast cells as prepared by section 5.1 and 5.2 can be propagated on a large scale to make the biological compositions of the invention. The method comprises culturing the yeast cells of the four yeast cell components separately in the presence of one or more EM fields for a period of time, diluting the growing yeast cells with fresh medium, and repeating the process. The method can be carried out as a batch process or a continuous process.

In one preferred embodiment, a set of three containers each comprising a set of electrodes for generating an electromagnetic field as described in section 5.1 and 5.2 are set up each with 1000 liters of a culture medium. The culture medium comprises nutrients assimilable by the yeast cells as shown in Table 5.

**Table 5**

| **Material** | **Quantity** |
|---|---|
| Wild hawthorn juice | 200 liters |
| Starch | 30 kg |
| peptone | 5 kg |
| Distilled water | 800 liters |

The wild hawthorn juice is an extract of fresh wild hawthorn fruits prepared by washing the fruits clean, drying the fruits to less than about 8% moisture, crushing the dried fruits to less than about 20 mesh, and mixing the crushed wild hawthorn with water at a ratio of 1 liters of water per 100 gram of crushed fruits. The mixture is then stirred continuously for 12 hours while the temperature is maintained at 28°C to 30°C. The mixture is then centrifuged at 1000 rpm to collect the supernatant which is used as described above. Preferably, the procedures are carried out under sterile conditions.

The first container(s) is inoculated with activated and conditioned yeast cells. The yeast cells in the first container is used as a seed culture to inoculate the culture medium in second container. About 5 ml of yeast cells (1 x 10⁸ cells/ml) in the first container is added per 100 ml of culture medium in the second container.

The yeast cells in the second container (6) are then subjected to one or more EM fields. For the activated and conditioned yeast cells of the first yeast cell component, the frequencies of 9211, 9217, 9222 and 9225 MHz are used. The field strength is in the range of 50 to 230 mV/cm, preferably at 120 mV/cm. The temperature is maintained at 36 ± 1 °C. The yeast culture is exposed to the EM field for about 42-134 hours.

For the activated and conditioned yeast cells of the second yeast cell component, EM fields of frequencies of 8137, 8140, 8144 and 8153 MHz are used. The field strength is in the range of 50 to 230 mV/cm preferably at 140 mV/cm. The temperature is maintained at 36 ± 2°C. The yeast culture is exposed to the EM field for about 48-140 hours.

For the activated and conditioned yeast cells of the third yeast cell component, EM fields of frequencies of 11311, 11317, 11321 or 11328 MHz are used. The field strength is in the range of 30 to 230 mV/cm preferably at 170 mV/cm. The temperature is maintained at 36 ± 1 °C. The yeast culture is exposed to the EM field for about 36-104 hours.

For the activated and conditioned yeast cells of the fourth yeast cell component, EM fields of frequencies of 17021, 17025, 17030, and 17037 MHz and the field strength is in the range of 30 to 230 mV/cm, preferably at 95 mV/cm. The temperature is maintained at 36 ± 2°C. The yeast culture is exposed to the EM field for about 38-112 hours.

After culturing in the second container, the yeast cells are transferred into the third container and further cultured in the presence of EM fields using the same set of EM field characteristics described above for culturing in the second container.

The yeast cell culture resulting from the end of this stage can be used directly as a biological composition of the invention, or mixed in equal proportions to form a preferred biological composition that comprises all four yeast cell components.

Preferably, a biological feed additive comprising all four yeast cell components is prepared by adsorbing the yeast cells to an absorbent carrier. To facilitate this, the yeast cell culture is concentrated using methods known in the art. The concentration process is carried out in two stages. At the first stage, the volume of the liquid culture is reduced to about 80% of the original volume. At the end of the second stage, the volume is reduced to about 50%. The biological feed additive comprising all four yeast cell components can be prepared by mixing yeast cells of each component with an absorbent carrier such as starch or zeolite powder (less than 200 mesh) at a ratio of 100 to 120 ml of concentrated yeast cells (5-10 kg dried cells) per 1000 kg of feed additive. For every 990 to 995 kg of carrier, 5 to 10 kg of the yeast and zeolite powder mixture was added. The mixture is dried at a temperature not exceeding 65 °C for a period of time less than 10 minutes such that the yeast cells become dormant, and the moisture content is below 5%. The final dried product comprises greater than or equal to about 10⁸ yeasts per gram. The final dried product can be used by mixing it with animal feed at a ratio of about 0.3 to 0.6%.

### 6. EXAMPLE

The following example illustrates the manufacture of a biological composition that can be used as an animal feed additive.

The biological composition comprises the following four components of yeasts: *Saccharomyces carlsbergensis* AS2.162, *Saccharomyces cerevisiae* AS2.558, AS2.14 and ACCC2036. Each component of yeast cells is capable of inhibiting the spread of FMD viruses and reducing the number of FMD viruses in culture. The four yeast cell components are prepared and tested separately as follows:

A starting culture containing about 10⁵ cells/ml of AS2.558 is placed into the container (2) as shown in Figure 1. The medium has the composition shown in Table 1. Initially, the yeast cells are cultured for about 24-56 hours at 33 ± 5 °C without an EM field. Then, in the same medium, at 33 ± 5 °C, the yeast cells are cultured in the presence of a series of four EM fields applied in the order stated: 9211MHz at 223mv/cm for 55 hrs; 9217MHz at 223mv/cm for 55 hrs; 9222MHz at 223mv/cm for 12 hrs; and 9225MHz at 223mv/cm for 12 hrs. The yeast cells were conditioned by further culturing in the gastric juice of a cattle and hawthorn juice as described in section 5.2, in the presence of a series of two EM fields: 9211 MHz at 223 mV/cm for 55 hours and 9217 MHz at 223 mV/cm for 55 hours. After the last culture period, the yeast cells are either used within 24 hours to make the biological compositions, or dried for storage as described in section 5.3.

The activated and conditioned yeast cells of the first yeast cell component was tested for their effect on FMD viruses in vitro. 300 ml of bovine serum was obtained from a cow (of a Dutch breed kept for meat) under sterile conditions. 0.15 ml of activated and conditioned AS 2.558 yeast cells (10⁹ cells per ml) were added to the bovine serum and the mixture was kept in a tissue culture incubator at 37°C under CO₂ for about 12 hours. One ml of FMD viruses (10¹² per ml) was added to the bovine serum - yeast cell mixture which was incubated for another 12 hours. The number of FMD viruses remaining in culture was counted. The experiment was repeated with a blank (i.e, no yeast cells were added), and with AS2.558 yeast cells which were neither activated nor conditioned. The results are shown in Table 6 below.

**Table 6:**

| Experimental Groups | Concentration of FMD virus after 12 hours of incubation | Concentration of FMD virus before incubation | Percentage change in concentration of FMD virus |
|---|---|---|---|
| Activated and Conditioned AS2.558 | 0.22 x 10⁹ /ml | 3 x 10⁹ /ml | 7.3% |
| Non-activated non-conditioned AS2.558 | 10.5 x 10⁹ /ml | 3 x 10⁹ /ml | 350% |
| No yeast cells | 13 x 10⁹ /ml | 3 x 10⁹ /ml | 433% |

To prepare the second component, a starting culture containing about 10⁵ cells/ml of AS2.182 is placed into the container (2) as shown in Figure 1. The medium has a composition as shown in Table 2. Initially, the yeast cells are cultured for about 24 to 56 hours at 33 ± 5°C without an EM field. Then, in the same medium, at 33 ± 5°C, the yeast cells are cultured in the presence of a series of four EM fields applied in the order stated: 8137MHz at 183mv/cm for 8 hrs; 8140MHz at 183mv/cm for 8 hrs; 8144MHz at 183mv/cm for 62 hrs; and 8153MHz at 183mv/cm for 62 hrs. The yeast cells were conditioned by further culturing in the gastric juice of a cattle and hawthorn juice as described in section 5.2, in the presence of a series of two EM fields: 8144 MHz at 183 mV/cm for 62 hours and 8153 MHz at 183 mV/cm for 62 hours. After the last culture period, the yeast cells are either used within 24 hours to make the biological compositions, or dried for storage as described in section 5.3.

The activated and conditioned yeast cells of the second yeast cell component was tested for their effect on FMD viruses in vitro. 300 ml of bovine serum was obtained from a cattle (of a Dutch breed kept for meat) under sterile conditions. 0.15 ml of activated and conditioned AS 2.182 yeast cells (10⁹ cells per ml) were added to the bovine serum and the mixture was kept in a tissue culture incubator at 37°C under CO₂ for about 12 hours. One ml of FMD viruses (10¹² per ml) was added to the bovine serum - yeast cell mixture which was incubated for another 12 hours. The number of FMD viruses remaining in culture was counted. The experiment was repeated with a blank (i.e, no yeast cells were added), and with AS2.182 yeast cells which were neither activated nor conditioned. The results are shown in Table 7 below.

**Table 7:**

| Experimental Groups | Concentration of FMD virus after 12 hours of incubation | Concentration of FMD virus before incubation | Percentage change in concentration of FMD virus |
|---|---|---|---|
| Activated and Conditioned AS2.182 | 0.25 x 10⁹ /ml | 3 x 10⁹ /ml | 8.3% |
| Non-activated non-conditioned AS2.182 | 12.3 x 10⁹ /ml | 3 x 10⁹ /ml | 410% |
| No yeast cells | 13 x 10⁹ /ml | 3 x 10⁹ /ml | 433% |

For the third yeast cell component, a starting culture containing about 10⁵ cells/ml of AS2.14 is placed into the container (2) as shown in Figure 1. The medium has a composition as shown in Table 3. Initially, the yeast cells are cultured for about 42 hours at 33 ± 5 °C without an EM field. Then, in the same medium, at 33 ± 5 °C, the yeast cells are cultured in the presence of a series of four EM fields applied in the order stated: 11311MHz at 192mv/cm for 10 hrs; 11317MHz at 192mv/cm for 10 hrs; 11321MHz at 192mv/cm for 42 hrs; and 11328MHz at 192mv/cm for 42 hrs. The yeast cells were conditioned by further culturing in the gastric juice of a cattle and hawthorn juice as described in section 5.2, in the presence of a series of two EM fields: 11321 MHz at 192 mV/cm for 42 hours and 11328 Hz at 192 mV/cm for 42 hours. After the last culture period, the yeast cells are either used within 24 hours to make the biological compositions, or dried for storage as described in section 5.3.

The activated and conditioned yeast cells of the third yeast cell component was tested for their effect on FMD viruses in vitro. 300 ml of bovine serum was obtained from a cattle (of a Dutch breed kept for meat) under sterile conditions. 0.15 ml of activated and conditioned AS 2.14 yeast cells (10⁹ cells per ml) were added to the bovine serum and the mixture was kept in a tissue culture incubator at 37 °C under CO₂ for about 12 hours. One ml of FMD viruses (10¹² per ml) was added to the bovine serum - yeast cell mixture which was incubated for another 12 hours. The number of FMD viruses remaining in culture was counted. The experiment was repeated with a blank (i.e, no yeast cells were added), and with AS2.14 yeast cells which were neither activated nor conditioned. The results are shown in Table 8 below.

**Table 8:**

| Experimental Groups | Concentration of FMD virus after 12 hours of incubation | Concentration of FMD virus before incubation | Percentage change in concentration of FMD virus |
|---|---|---|---|
| Activated and Conditioned AS2.14 | 0.17 x 10⁹ /ml | 3 x 10⁹ /ml | 5.7% |
| Non-activated non-conditioned AS2.14 | 12.7 x 10⁹ /ml | 3 x 10⁹ /ml | 423% |
| No yeast cells | 13 x 10⁹ /ml | 3 x 10⁹ /ml | 433% |

To prepare the fourth component, a starting culture containing about 10⁵ cells/ml of ACCC2036 is placed into the container (2) as shown in Figure 1. The medium has a composition as shown in Table 4. Initially, the yeast cells are cultured for about 52 hours at 33 ± 5°C without an EM field. Then, in the same medium, at 33 ± 5°C, the yeast cells are cultured in the presence of a series of four EM fields applied in the order stated: a 17021MHz at 211mv/ for 55 hrs; 17025MHz at 211mv/cm for 55 hrs; 17030MHz at 211mv/ and for 10 hrs; 17037MHz at 211mv/ for 10 hrs. The yeast cells were conditioned by further culturing in the gastric juice of a cattle and hawthorn juice as described in section 5.2, in the presence of a series of two EM fields: 17021MHz at 211 mV/cm for 55 hours and 17025 MHz at 211 mV/cm for 55 hours. After the last culture period, the yeast cells are either used within 24 hours to make the biological compositions, or dried for storage as described in section 5.3.

The activated and conditioned yeast cells of the fourth yeast cell component was tested for their effect on FMD viruses in vitro. 300 ml of bovine serum was obtained from a cattle (of a Dutch breed kept for meat) under sterile conditions. 0.15 ml of activated and conditioned ACCC2036 yeast cells (10⁹ cells per ml) were added to the bovine serum and the mixture was kept in a tissue culture incubator at 37°C under CO₂ for about 12 hours. One ml of FMD viruses (10¹² per ml) was added to the bovine serum - yeast cell mixture which was incubated for another 12 hours. The number of FMD viruses remaining in culture was counted. The experiment was repeated with a blank (i.e, no yeast cells were added), and with ACCC2036 yeast cells which were neither activated nor conditioned. The results are shown in Table 9 below.

**Table 9:**

| Experimental Groups | Concentration of FMD virus after 12 hours of incubation | Concentration of FMD virus before incubation | Percentage change in concentration of FMD virus |
|---|---|---|---|
| Activated and Conditioned ACCC2036 | 0.19 x 10⁹ /ml | 3 x 10⁹ /ml | 6.3% |
| Non-activated non-conditioned ACCC2036 | 11.7 x 10⁹ /ml | 3 x 10⁹ /ml | 390% |
| No yeast cells | 13 x 10⁹ /ml | 3 x 10⁹ /ml | 433% |

The activated and conditioned yeast cells of all four yeast cell components were tested together for their effect on FMD viruses in vitro. 300 ml of bovine serum was obtained from a cattle (of a Dutch breed kept for meat) under sterile conditions. 0.15 ml of activated and conditioned yeast cells (10⁹ cells per ml) from each of the four yeast cell components as described above (AS2.558, AS2.162, AS2.14 and ACCC2036) were added to the bovine serum and the mixture was kept in a tissue culture incubator at 37°C under CO₂ for about 12 hours. One ml of FMD viruses (10¹² per ml) was added to the bovine serum-yeast cell mixture which was incubated for another 12 hours. The number of FMD viruses remaining in culture was counted. The experiment was repeated with a blank (i.e, no yeast cells were added), and with the yeast cells which were neither activated nor conditioned. The results are shown in Table 10 below.

**Table 10:**

| Experimental Groups | Concentration of FMD virus after 12 hours of incubation | Concentration of FMD virus before incubation | Percentage change in concentration of FMD virus |
|---|---|---|---|
| Activated and Conditioned yeast cells | undetectable | 3 x 10⁹ /ml | n.a. |
| Non-activated non-conditioned yeast cells | 9.7 x 10⁹ /ml | 3 x 10⁹ /ml | 323.3% |
| No yeast cells | 13.5 x 10⁹ /ml | 3 x 10⁹ /ml | 450% |

The present invention is not to be limited in scope by the specific embodiments described which are intended as single illustrations of individual aspects of the invention, and functionally equivalent methods and components are within the scope of the invention. Indeed various modifications of the invention, in addition to those shown and described herein will become apparent to those skilled in the art from the foregoing description and accompanying drawings. Such modifications are intended to fall within the scope of the appended claims.

## Claims

1. A biological composition comprising at least one of the following yeast cell components:
(a) a first yeast cell component comprising a plurality of yeast cells that are prepared by culturing the yeast cells in an electromagnetic field or a series of electromagnetic fields having a frequency in the range of 9208 to 9230 MHz and a field strength of 50 to 250 mV/cm;
(b) a second yeast cell component comprising a plurality of yeast cells that are prepared by culturing the yeast cells in an electromagnetic field or a series of electromagnetic fields having a frequency in the range of 8135 to 8155 MHz and a field strength of 45 to 220 mV/cm;
(c) a third yeast cell component comprising a plurality of yeast cells that are prepared by culturing the yeast cells in an electromagnetic field or a series of electromagnetic fields having a frequency in the range of 11310 to 11330 MHz and a field strength of 63 to 220 mV/cm; and
(d) a fourth yeast cell component comprising a plurality of yeast cells that are prepared by culturing the yeast cells in an electromagnetic field or a series of electromagnetic fields having a frequency in the range of 17020 to 17040 MHz and a field strength of 50 to 230 mV/cm.

2. The biological composition of claim 1 which comprises the yeast cell components of (a), (b), (c) and (d).

3. The biological composition of claim 1 or 2, wherein the yeast cells are cells of Saccharomyces.

4. The biological composition of claim 1 or 2, wherein the yeast cells are cells of Saccharomyces cerevisiae or Saccharomyces carlsbergensis.

5. The biological composition of claim 1 or 2 in which the yeast cells are dried.

6. An animal feed composition comprising (i) the biological composition of claim 1 or 2, and (ii) cattle feed or swine feed.

7. The animal feed composition of claim 6 wherein the biological composition further comprises zeolite powder at a ratio of about 10⁹ yeast cells to 1 g of zeolite powder.

8. The animal feed composition of claim 7 in which 0.5% to 1% by weight is the biological composition of claim 1 or 2.

9. A method for preparing a biological composition, said method comprising culturing a plurality of yeast cells in an electromagnetic field or a series of electromagnetic fields having a frequency in the range of 9208 to 9230 MHz and a field strength of 50 to 250 mV/cm.

10. The method of claim 9, wherein said method further comprises culturing the plurality of yeast cells in one or more of the electromagnetic fields in a culture medium comprising gastric juice of cattle or swine, and wild hawthorn juice.

11. A method for preparing a biological composition, said method comprising culturing a plurality of yeast cells in an electromagnetic field or a series of electromagnetic fields having a frequency in the range of 8135 to 8155 MHz and a field strength of 45 to 220 mV/cm.

12. The method of claim 11, wherein said method further comprises culturing the plurality of yeast cells in one or more of the electromagnetic fields in a culture medium comprising gastric juice of cattle or swine, and wild hawthorn juice.

13. A method for preparing a biological composition, said method comprising culturing a plurality of yeast cells in an electromagnetic field or a series of electromagnetic fields having a frequency in the range of 11310 to 11330 MHz and a field strength of 63 to 220 mV/cm.

14. The method of claim 13, wherein said method further comprises culturing the plurality of yeast cells in one or more of the electromagnetic fields in a culture medium comprising gastric juice of cattle or swine, and wild hawthorn juice.

15. A method for preparing a biological composition, said method comprising culturing a plurality of yeast cells in an electromagnetic field or a series of electromagnetic fields having a frequency in the range of 17020 to 17040 MHz and a field strength of 50 to 230 mV/cm.

16. The method of claim 15, wherein said method further comprises culturing the plurality of yeast cells in one or more of the electromagnetic fields in a culture medium comprising gastric juice of cattle or swine, and wild hawthorn juice.

17. A method of making an animal feed composition, said method comprising
(a) preparing one or more of the yeast cell components of claim 1,
(b) adsorbing the yeast cell components of (a) to a carrier, and
(c) mixing the dried yeast cells and carrier with cattle feed or swine feed.

18. The method of claim 17, wherein the adsorbing step comprises (i) concentrating the yeast cell culture by about 50%, (ii) mixing the yeast cell culture with the carrier; and (iii) drying the mixture at a temperature not exceeding 65 °C to reduce the moisture content to below 5%.

19. A method for reducing the incidence of foot-and-mouth disease in a cloven-hooved animal comprising feeding the ruminant for a period of time an animal feed composition comprising at least one of the following yeast cell components:
(a) a first yeast cell component comprising a plurality of yeast cells that are prepared by culturing the yeast cells in an electromagnetic field or a series of electromagnetic fields having a frequency in the range of 9208 to 9230 MHz and a field strength of 50 to 250 mV/cm;
(b) a second yeast cell component comprising a plurality of yeast cells that are prepared by culturing the yeast cells in an electromagnetic field or a series of electromagnetic fields having a frequency in the range of 8135 to 8155 MHz and a field strength of 45 to 220 mV/cm;
(c) a third yeast cell component comprising a plurality of yeast cells that are prepared by culturing the yeast cells in an electromagnetic field or a series of electromagnetic fields having a frequency in the range of 11310 to 11330MHz and afield strength of 63 to 220 mV/cm; and
(d) a fourth yeast cell component comprising a plurality of yeast cells that are prepared by culturing the yeast cells in an electromagnetic field or a series of electromagnetic fields having a frequency in the range of 17020 to 17040 MHz and a field strength of 50 to 230 mV/cm.

20. The method of claim 19, wherein the animal feed composition comprises the yeast cell components of (a), (b), (c) and (d), and a carrier which is starch or zeolite powder.

21. The method of claim 19, wherein said yeast cells are *Saccharomyces carlsbergensis* or *Saccharomyces cerevisiae* cells.

22. The method of claim 19, wherein the yeast cell components and zeolite powder comprises 0.5% by weight of the animal feed composition.

23. The composition of claim 1 or 2, wherein the plurality of yeast cells used in preparing the first yeast cell component comprise cells of *Saccharomyces cevervisae* AS2.558, wherein the plurality of yeast cells used in preparing the second yeast cell component comprise cells of *Saccharomyces carlsbergensis* AS2.162, wherein the plurality of yeast cells used in preparing the third yeast cell component comprise cells of *Saccharomyces cevervisae* AS2.14, and wherein the plurality of yeast cells used in preparing the fourth yeast cell component comprise cells of *Saccharomyces cevervisae* ACCC2036.

24. The animal feed composition of claim 6, wherein the plurality of yeast cells used in preparing the first yeast cell component comprise cells of *Saccharomyces cevervisae* AS2.558, wherein the plurality of yeast cells used in preparing the second yeast cell component comprise cells of *Saccharomyces carlsbergensis* AS2.162, wherein the plurality of yeast cells used in preparing the third yeast cell component comprise cells of *Saccharomyces cevervisae* AS2.14, and wherein the plurality of yeast cells used in preparing the fourth yeast cell component comprise cells of *Saccharomyces cevervisae* ACCC2036.

25. The method of claim 19, wherein the animal is a cow, a swine, a sheep, a goat, a deer, a buffalo, a bison, a camel, a llama, a dromedary or a vicuna.
